# EUROPÄISCHE PATENTSCHRIFT

(11) **EP 0 545 208 B1**
(45) Veröffentlichungstag und Bekanntmachung des Hinweises auf die Patenterteilung: **06.03.1996**
(21) Anmeldenummer: 92119929.5
(22) Anmeldetag: 24.11.1992
(51) Int. Cl.: C07C 291/04, C07C 209/84

(54) **Verfahren zur Herstellung tertiärer Aminoxide**
Process for the preparation of tertiary amine oxides
Procédé pour la préparation des amine oxides tertiaires

(30) Priorität: 06.12.1991 DE 4140259
(43) Veröffentlichungstag der Anmeldung: 09.06.1993
(73) Patentinhaber: BASF Aktiengesellschaft, D-67063 Ludwigshafen (DE)
(72) Erfinder: Koehler, Ulrich, Dr., W-6800 Mannheim 51 (DE); Siegel, Hardo, Dr., W-6720 Speyer (DE); Seybold, Guenther, Dr., W-6708 Neuhofen (DE)

(56) Entgegenhaltungen:
- EP-A- 0 320 694
- EP-A- 0 356 918
- EP-A- 0 413 259
- FR-A- 1 556 993
- R. SCHRÖTER, Houben-Weyl, Methoden der Organischen Chemie, 4. Aufl. Bd XI/1', E. Müller, Stuttgart, DE, S. 1029-1033

## Beschreibung

Die vorliegende Erfindung betrifft ein Verfahren zur Herstelung tertiärer Aminoxide mit einem geringen Gehalt an Nitrosaminen durch die Umsetzung eines tertiären Amins mit einer wäßrigen Wasserstoffperoxidlösung.

Tertiäre Aminoxide können nach dem Verfahren von DE-A 16 94 048, durch die Umsetzung der betreffenden tertiären Amine mit einer wäßrigen Wasserstoffperoxidlösung hergestellt werden und dienen z.B. als Lösungsmittel für Cellulose, Polyamide und Polypeptide bei der Faserherstellung. Des weiteren werden tertiäre Aminoxide als Cooxidationsmittel zur Herstellung von vicinalen Diolen aus Olefinen mittels Osmiumtetroxid verwendet (vgl. Org. Synth. 58, 43 (1978)). Von besonderer Bedeutung ist für die genannten Verwendungszwecke N-Methylmorpholin-N-oxid. Da tertiäre Aminoxide nur mäßig lagerstabil sind und als reine Stoffe zur Sauerstoffabspaltung neigen, werden sie üblicherweise in Form ihrer Lösungen, im allgemeinen in Form ihrer wäßrigen Lösungen gelagert und vertrieben. Beispielsweise wird N-Methylmorpholin-N-oxid in der Regel als 50 bis 60 gew.-%ige wäßrige Lösung gehandelt.

Ein Problem bei der Anwendung der tertiären Aminoxide ist deren Verunreinigung mit Nitrosaminen, welche als kanzerogen gelten. Es hat deshalb nicht an Versuchen gefehlt, Verfahren zur Herstellung von nitrosaminarmen tertiären Aminoxiden zu entwickeln. FR-A 26 32638 beschreibt die inhibierende Wirkung von Kohlendioxid auf die Nitrosaminbildung bei der Herstellung der tertiären Aminoxide, in EP-A 320 694 wird allerdings festgestellt, daß ein Zusatz von Kohlendioxid für eine zufriedenstellende Reduzierung der Nitrosaminbildung allein nicht ausreicht und daher zusätzlich noch das Reduktionsmittel Ascorbinsäure zur Reaktionsgemisch gegeben werden muß. Gemäß EP-A 356 918 soll die Herstellung von tertiären Aminoxiden in Gegenwart von Kohlendioxid und metallischem Titan zu tertiären Aminoxiden mit einem vermindertem Gehalt an Nitrosaminen führen. Die beiden letztgenannten Verfahren haben den Nachteil, daß zum einen der Reaktionsmischung ein teurer Hilfsstoff - Ascorbinsäure - zugesetzt werden muß, welcher nicht mehr zurückgewonnen werden kann und zum anderen, aufgrund der Verwendung metallischen Titans zur Verminderung des Nitrosamingehaltes, das Verfahren im großtechnischen Maßstab verfahrenstechnisch aufwendiger gestaltet werden muß. Dadurch werden beide Verfahren erheblich verteuert.

Gemäß US-A 4 247 480 werden tert. Aminoxide durch die Oxidation von tert. Aminen mit Wasserstoffperoxid in Gegenwart von Kohlendioxid und Chelatbildnern, wie Diethylentriaminpentaessigsäure, Polyphosphorsäuren und Polycarbonsäuren, hergestellt, wobei die Chelatbildner eine Zersetzung des Wasserstoffperoxids durch Schwermetallspuren verhindern sollen.

In EP-A 307 184 wird die Absenkung der Reaktionstemperatur zum Zwecke der Verringerung der Nitrosaminbildung bei der Herstellung der tertiären Aminoxide in Gegenwart von Kohlendioxid vorgeschlagen. Infolge der mit dieser Temperaturabsenkung verbundenen Verlangsamung der Reaktionsgeschwindigkeit ist auch dieses Verfahren wegen zu geringer Raum-Zeit-Ausbeuten unwirtschaftlich. Darüber hinaus besteht bei diesem Verfahren die Gefahr einer gefährlichen Akkumulation von Wasserstoffperoxid im Reaktor.

Laut EP-A 426 084 führt die Anwendung von Kohlendioxid zudem zu einer Verfärbung des tert. Aminoxid-Produktes, zu deren Vermeidung das Arbeiten unter einer Inertgasatmosphäre helfen soll, wodurch das Verfahren wiederum verteuert wird.

In EP-A 413 259 wird ein Verfahren zur Verminderung des Gehaltes an primärem und sekundärem Amin in einem tertiären Amin beschrieben, wobei das zu behandelnde tertiäre Amin mit einem organischen Anhydrid in Kontakt gebracht wird, bis die angestrebte Verminderung des Gehaltes an primärem und sekundärem Amin erreicht ist.

Der vorliegenden Erfindung lag daher die Aufgabe zugrunde, ein einfaches und wirtschaftliches Verfahren zur Herstellung von tert. Aminoxiden mit einem niedrigen Gehalt an Nitrosaminen zur Verfügung zu stellen.

Dementsprechend wurde ein Verfahren zur Herstellung tert. Aminoxide mit einem Nitrosamingehalt unter 50 ppb durch die Umsetzung eines tertiären Amins mit einer wäßrigen Wasserstoffperoxidlösung gefunden, das dadurch gekennzeichnet ist, daß man als Ausgangsmaterial tertiäre Amine verwendet, deren Gehalt an primären und sekundären Aminen insgesamt nicht mehr als 0,05 Gew.-% beträgt.

Tert. Amine mit einem Gehalt an primären und sekundären Aminen von weniger als 0,05 Gew.-%, vorzugsweise von weniger als 0,02 Gew.-% können aus den betreffenden tert. Aminen, welche diese primären und sekundären Amine herstellungsbedingt enthalten, z.B. destillativ erhalten werden. Um den gegebenenfalls hohen Aufwand einer solchen Destillation zu verringern, ist es zweckmäßig, dem verunreinigten tertiären Amin Abfangreagenzien zuzusetzen, welche selektiv mit den primären und sekundären Aminen reagieren.Die so gebildeten Verbindungen können im derart behandelten tertiären Amin verbleiben, ohne daß dadurch die nachfolgende Erzeugung des tertiären Aminoxids mit Hilfe eines Peroxids, insbesondere mit Wasserstoffperoxid, gestört wird. Gewünschtenfalls können diese Abfangreagenzien und ihre bei der Reaktion mit den primären und/oder sekundären Aminen gebildeten Derivate destillativ, extraktiv, durch Filtration oder Zentrifugation, vom tertiären Amin abgetrennt werden.

Grundsätzlich können zu diesem Zwecke solche Substanzen als Abfangreagenzien eingesetzt werden, die mit primären und sekundären Aminen schneller reagieren als mit tertiären Aminen und deren Umsetzung mit diesen Aminen nach möglichst kurzer Zeit möglichst vollständig verläuft. Als Abfangreagenzien für primäre und sekundäre Amine können deshalb alle Reagenzien besonders vorteilhaft eingesetzt werden, die in der organischen Synthesechemie zum Schutz, d.h. zur Blockierung der Aminfunktion, bei der Synthese organischer Moleküle benutzt werden. Solche Reagenzien zur Einführung sogenannter Schutzgruppen an primäre und/oder sekundäre Amine werden in T. Greene, Protective Groups in Organic Synthesis, S. 223-287, Wiley Interscience, New York 1981 beschrieben.

Beispielhaft für derartige Reagenzien, die zur Entfernung primärer und sekundärer Amine aus tertiären Aminen eingesetzt werden können, seien die folgenden Verbindungsklassen genannt: Halogenformiate, Halogenformamide, Carbonsäureanhydride, Acylhalogenide, Carbonsäureester, Ketene und deren Dimere, Phosgen, Kohlensäureester, Pyrokohlensäureester, Isocyanate, Phosphinsäurehalogenide, Phosphonsaürehalogenide, Phosphorylhalogenide,Sulfensäurehalogenide, Sulfonsäurehalogenide, Sulfonsäureester oder -anhydride.

Werden Halogenformiate der Formel I 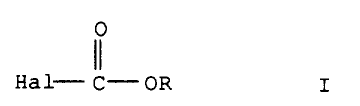 zum Abfangen der primären und/oder sekundären Amine eingesetzt, bilden sich die betreffenden Carbaminsäureester IIa 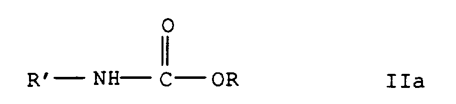 und/oder IIb 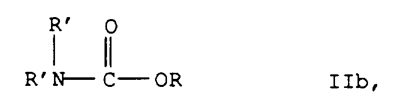 welche vom tert. Amin, das mit diesen Reagenzien nicht reagiert, leicht durch Destillation abgetrennt werden können. Als Halogenformiate können grundsätzlich alle Arten von Halogenformiaten verwendet werden, also solche, in denen Hal ein Fluor-, Chlor-, Brom- oder Jodatom ist und in denen R einen beliebigen aliphatischen oder aromatischen Rest bezeichnen kann, aufgrund ihrer einfachen Herstellung und insbesondere aus Kostengründen werden allerdings vorzugsweise Chlorameisensäurealkylester und besonders bevorzugt Chlora-Chlorameisensäure-C₁- bis C₄-alkylester verwendet. Diese Verbindungen lassen sich auf einfache Weise durch die Umsetzung von Phosgen mit den betreffenden Alkoholen, beispielsweise nach Matzner et al, Chem. Rev. 64, 645 (1964) herstellen.

Anstelle der Halogenformiate können auch Halogenformamide der Formel III 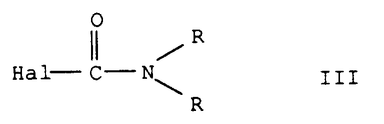 als Aminfänger verwendet werden. Bei der Umsetzung der Halogenformamide mit primären bzw. sekundären Aminen bilden sich die entsprechenden Harnstoffe der Formel IVa 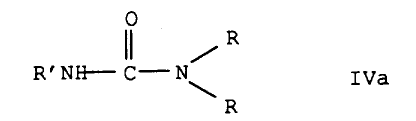 bzw. IVb 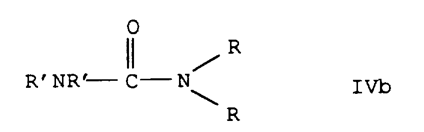 von denen das tert. Amin problemlos abdestilliert werden kann. Ebenso wie bei den Halogenformiaten können prinzipiell sämtliche Halogenformamide für diese Umsetzung eingesetzt werden, aufgrund ihrer einfachen Herstellung und kostengünstigen Verfügbarkeit werden allerdings Chlordialkylformamide, insbesondere Chlordi-C₁- bis C₄-alkylformamide bevorzugt. Diese Chlordialkylformamide können nach dem allgemein anwendbaren Verfahren von Rudenko et al, J. Gen. Chem. (USSR) 17, 2256 (1947) - Chemical Abstracts 47, 4918 durch die Umsetzung von Phosgen mit Dialkylaminen erhalten werden.

Carbonsäureanhydride reagieren mit den primären und sekundären Aminen unter Bildung der betreffenden Carbonsäureamide, von denen das tert. Amin leicht destillativ abgetrennt werden kann. Grundsätzlich läßt sich jedes Carbonsäureanhydrid zur Entfernung der unerwünschten Amine aus dem tert. Amin einsetzen, besonders bevorzugt werden jedoch Phthalsäureanhydrid sowie die offenkettigen oder cyclischen Anhydride der C₂- bis C₄-Mono- und/oder Dicarbonsäuren, namentlich Acetanhydrid, Propionsäureanhydrid, Maleinsäureanhydrid und Bernsteinsäureanhydrid verwendet.

Ebenso wie die Carbonsäureanhydride bilden die Acylhalogenide bei der Umsetzung mit primären und sekundären Aminen Carbonsäureamide. Auch hier können generell alle Acylhalogenide zur Acylierung der primären und sekundären Amine verwendet werden, doch sind aus Gründen der Wirtschaftlichkeit Acylhalogenide einfacher, preiswerter Carbonsäuren, insbesondere C₂- bis C₄-Acylhalogenide, wie Acetyl-, Propionyl- und Butyrylhalogenide sowie Benzoyl- und Phthaloylhalogenide bevorzugt. Es können sowohl Acylfluoride, Acylchloride, Acylbromide als auch Acyljodide verwendet werden, aus Kostengründen werden zweckmäßigerweise Acylchloride verwendet.

Auch Carbonsäureester können zur Acylierung der primären und sekundären Amine verwendet werden. Auch bei dieser Acylierungsmethode besteht grundsätzlich keine Beschränkung bezüglich des zu verwendenden Carbonsäureesters. Bevorzugte Carbonsäureester sind aus Kostengründen die Ester der Benzoesäure, Phthalsäure sowie die Ester der C₁- bis C₆-Mono- und Dicarbonsäuren, insbesondere die Ester der Ameisensäure, Essigsäure, Propionsäure, Maleinsäure, Bernsteinsäure und Adipinsäure. Selbstverständlich können auch innere Ester von Carbonsäuren, also Lactone, zur Acylierung der primären und sekundären Amine herangezogen werden. Ein bevorzugtes Lacton für diesen Zweck ist γ-Butyrolacton.

Die Carbonsäureester, Carbonsäureanhydride und Acylhalogenide können nach den Standardverfahren von Organikum, Organisch-chemisches Grundpraktikum, 17. Auflage, S. 402-408 und 419-424 hergestellt werden. Maleinsäureanhydrid und Phthalsäureanhydrid sind Grundchemikalien und werden großtechnisch beispielsweise durch die partielle Oxidation von Butan oder Buten bzw. Xylol nach den in Weissermel, Arpe: Industrielle Organische Chemie, S. 343-349 bzw. 359-365, Verlag Chemie, Weinheim 1978, beschriebenen Verfahren produziert. Die Hydrierung der Doppelbindung im Maleinsäureanhydrid führt zu Bernsteinsäureanhydrid, dessen weitere Hydrierung zu γ-Butyrolacton.

Zur Umwandlung der primären und sekundären Amine in leicht abtrennbare Carbonsäureamide können vorteilhaft auch die Ketene der Formel V 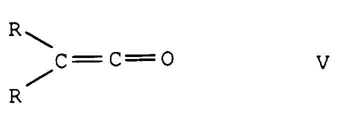 in der die Reste R gleich oder verschieden sein können und für Wasserstoff, Aryl-, insbesondere für Phenyl- und/oder für C₁- bis C₂₀-Alkylgruppen stehen können, oder deren cyclische Dimere der Formel VI 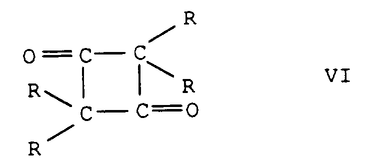 angewandt werden. Die Ketene können z.B. aus den entsprechenden Carbonsäuren durch Wasserabspaltung oder aus den entsprechenden Acylhalogeniden durch die Eliminierung von Halogenwasserstoff beispielsweise gemäß den Verfahren von J. March, Advanced Organic Chemistry, 3. Auflage, S. 902+916, Wiley, New York 1985 erzeugt werden. Sie sind allesamt sehr reaktiv und addieren primäre und sekundäre Amine spontan unter Amidbildung. Besonders bevorzugt wird das aus Essigsäure erhältliche Keten Va

H₂C=C=O Va

und sein cyclisches Dimeres verwendet.

Auch Phosgen und sein schwefelhaltiges Analogon, das Thiophosgen, eignen sich aufgrund ihrer hohen Reaktivität zum Abfangen der primären und sekundären Amine, wobei sich schwerflüchtige Harnstoff- bzw. Thioharnstoffderivate bilden, von denen das tert. Amin leicht abdestilliert werden kann.

Mit Kohlensäureestern der Formel VII 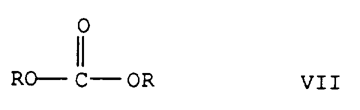 und Pyrokohlensäureestern der Formel VIII 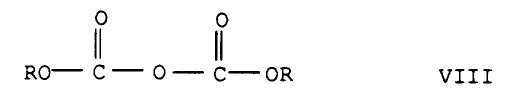 reagieren die primären und sekundären Amine zu Carbaminsäureestern IIa bzw. IIb. Auch hier lassen sich prinzipiell alle Kohlensäure- und Pyrokohlensäureester einsetzen, vorzugsweise werden jedoch die preiswerten Phenyl-, Benzyl- und C1- bis C6-Alkylderivate verwendet. Die Kohlensäureester VII sind z.B. nach dem Verfahren von DE-A 27 43 690, Pyrokohlensäureester z.B. nach dem Verfahren gemäß Howe et al, J. Org. Chem. 27, 1901 (1962) erhältlich. Es können selbstverständlich auch cyclische Kohlensäureester, wie Propylencarbonat, zum Abfangen der primären und sekundären Amine benutzt werden.

Schwerflüchtige Harnstoffe bilden sich bei der Umsetzung der primären und sekundären Amine mit Isocyanaten IX

R-N=C=O IX

wobei zweckmäßigerweise solche preiswert verfügbaren Isocyanate wie sie als Zwischenprodukte bei der Herstellung von Pflanzenschutzmitteln oder als Monomere bei der Herstellung von Polyurethanen und Polyharnstoffen verwendet werden, benutzt werden. Stellvertretend für solche Isocyanate seien Toluylendiisocyanat, Hexamethylendiisocyanat, 4,4'-Methylendi(phenylisocyanat), Isophorondiisocyanat, C₁- bis C₆-Alkylisocyanate, Phenylisocyanat und Tolylisocyanat genannt.

Geeignete Abfangreagenzien für primäre und sekundäre Amine sind weiterhin Phosphinigsäurehalogenide der Formel Xa 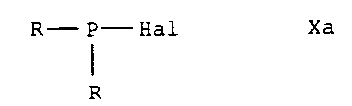 und Phosphinylhalogenide der Formel Xb 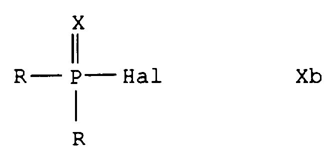 Phosphonigsäuredihalogenide und Phosphonigsäureesterhalogenide der Formel XI 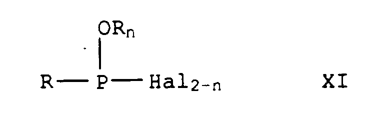 in der n 0 oder 1 bedeutet, die Phosphonylhalogenide der Formel XII 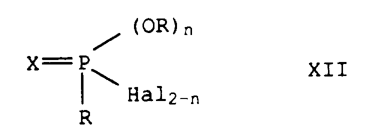 in der n die obengenannte Bedeutung hat und die Phosphorigsäureesterhalogenide der Formel XIII 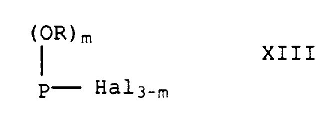 in der m für 0, 1 oder 2 steht, sowie Phosphorylhalogenide der Formel XIV 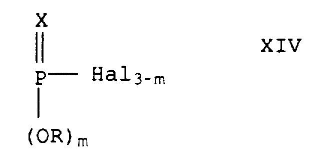 in der m die obengenannte Bedeutung hat. Der Rest X kann in diesen phosphorhaltigen Abfangreagenzien ein Schwefel- oder vorzugsweise ein Sauerstoffatom sein. Die Reste R können an sich beliebig gewählt werden, aufgrund ihrer preiswerten Verfügbarkeit werden solche Abfangreagenzien bevorzugt eingesetzt, in denen die Reste R C₁- bis C₆-Alkylgruppen oder Phenylgruppen sind. Auch bei den phosphorhaltigen Abfangreagenzien kann das oder die Halogenatome frei gewählt werden, in der Regel werden aber bevorzugt die betreffenden Chlorverbindungen verwendet. Phosphinigsäurehalogenide Xa und Phosphinylhalogenide Xb können gemäß den in Houben-Weyl, Methoden der Organischen Chemie, Band E1, S. 245ff, Thieme, Stuttgart 1982 und Band E2, S. 150ff, Thieme, Stuttgart 1982 beschriebenen Verfahren, Phosphonigsäureesterhalogenide XI nach den Verfahren von Houben-Weyl, Band E1, S. 273ff erhalten werden. Phosphonylhalogenide XII können gemäß Houben-Weyl, Band E2, S. 315ff und Phosphorigsäureesterhalogenide XIII nach dem Verfahren von Houben-Weyl, Band E1, S. 313ff hergestellt werden. Phosphorylhalogenide XIV sind nach dem Verfahren von Houben-Weyl, Band E2, S. 487ff erhältlich. Alle diese phosphorhaltigen Halogenverbindungen reagieren mit dem primären und sekundären Aminen zu den betreffenden entsprechenden Amiden.

Vorteilhaft anwendbare Reagenzien zur selektiven Derivatisierung der primären und sekundären Amine sind weiterhin Sulfonsäurehalogenide der Formel XV 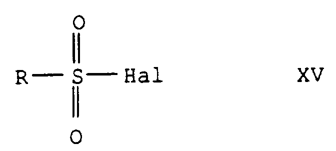 welche bei der Umsetzung mit diesen die schwerflüchtigen Sulfonamide bilden. Hal kann in den Sulfonsäurehalogeniden Fluor, Chlor oder Brom sein, vorzugsweise werden Sulfonsäurechloride verwendet. Zweckmäßigerweise werden solche Sulfonsäurehalogenide als Abfangreagenzien eingesetzt, die preiswert auf dem Markt erhältlich sind, insbesondere Methansulfonsäurechlorid, Ethansulfonsäurechlorid, Benzolsulfonsäurechlorid, Toluolsulfonsäurechlorid und Naphthalinsulfonsäurechlorid. Anstelle der Sulfonsäurehalogenide können auch die Ester, insbesondere die C₁- bis C₆-Alkyl- und Phenylester der Sulfonsäure zum Abfangen der im tert. Amin enthaltenen primären und sekundären Amine verwendet werden. Derartige Sulfonsäurechloride und Sulfonsäureester können z.B. gemäß den Verfahren von Organikum, Organisch-chemisches Grundpraktikum, 17. Aufl., S. 170-171, 307-313, 545-547 und 557-561, VEB Deutscher Verlag der Wissenschaften, Berlin 1988 hergestellt werden.

In gleicher Weise wie die Sulfonsäurehalogenide können auch Sulfensäurehalogenide der Formel XVI

R-S-Hal XVI

oder Sulfinsäurehalogenide der Formel XVII 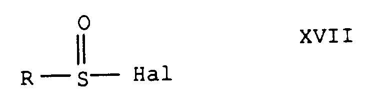 verwendet werden, doch sind diese aufgrund ihres in der Regel höheren Preises im allgemeinen nicht so bevorzugt.

Werden erfindungsgemäß zur Herstellung tert. Aminoxide tert. Amine als Ausgangsmaterialien benutzt, deren Gehalt an primären und sekundären Amine durch eines der genannten Behandlungsverfahren auf weniger als 0,05 Gew.-%, vorzugsweise auf weniger als 0,02 Gew.-% reduziert worden ist, so wird der Nitrosamingehalt der auf diese Weise hergestellten tert. Aminoxide drastisch reduziert.

Bei der besonders bevorzugten Verkörperung des erfindungsgemäßen Verfahrens werden die als Ausgangsmaterialien zur Herstellung der tert. Aminoxide verwendeten tert. Amine nicht in einer separaten Stufe nach einer der beschriebenen Behandlungsmethoden von den darin enthaltenen primären und sekundären Aminen abgetrennt, sondern es werden die Abfangreagenzien für die primären und sekundären Amine dem Reaktionsgemisch zur Umsetzung der tert. Amine zu den tert. Aminoxiden zugesetzt und mit den tert. Aminen vor der Zugabe des Oxidationsmittels inkubiert. Bei dieser Verkörperung des erfindungsgemäßen Verfahrens werden im wesentlichen nitrosaminfreie tert. Aminoxide erhalten.

Bei dieser bevorzugten Verkörperung des erfindungsgemäßen Verfahrens können alle obengenannten Abfangreagenzien für primäre und sekundäre Amine eingesetzt werden. Besonders bevorzugt werden bei dieser Verfahrensvariante aber Carbonsäurehalogenide, insbesondere C₁- bis C₄-Carbonsäurehalogenide und Benzoylhalogenide, vorzugsweise deren Chloride, Carbonsäureanhydride, insbesondere C₂- bis C₄-Carbonsäureanhydride und Phthalsäureanhydrid, Ketene und deren Dimere, insbesondere C₂- bis C₄-Ketene und deren Dimere, Sulfonsäurehalogenide, insbesondere C₁- bis C₄-Sulfonsäurehalogenide sowie aromatische Sulfonsäurehalogenide wie Benzolsulfonsäurehalogenide und Toluolsulfonsäurehalogenide, vorzugsweise deren Chloride sowie Phosphorylhalogenide, insbesondere Phosphorylchloride, verwendet. Als besonders bevorzugte Abfangreagenzien seien namentlich Acetylchlorid, Acetanhydrid, Keten, Phthalsäureanhydrid, Maleinsäure- und Bernsteinsäureanhydrid, Methansulfonsäurechlorid, Benzolsulfonsäurechlorid, Toluolsulfonsäurechlorid, Dimethoxyphosphorylchlorid und Phosphorylchlorid genannt.

Die Abfangreagenzien können bezüglich der im tertiären Amin enthaltenen primären und sekundären Amine in stöchiometrischen Mengen angewandt werden, zweckmäßigerweise werden die Abfangreagenzien aber im Überschuß bezüglich der abzutrennenden Amine zugesetzt. Im allgemeinen wird das 1,1- bis 10-fache, vorzugsweise das 1,5- bis 3-fache der stöchiometrisch benötigte Menge des jeweiligen Abfangreagenzes zur Entfernung der primären und sekundären Amine zugesetzt. Diese Mengenangaben gelten sowohl für die Verfahrensvariante bei denen die primären und sekundären Amine in einer separaten Stufe entfernt werden, als auch für die Verfahrensverkörperung bei der die unerwünschten Amine im Reaktionsgefäß zur Aminoxidherstellung vor der Zugabe des Oxidationsmittels abgefangen werden. Selbstverständlich können auch größere Überschüsse an Abfangreagenz zugegeben werden. Zur einfacheren Bemessung der Menge des zuzusetzenden Abfangreagenzes kann der Gehalt des tert. Amin-Ausgangsmaterials an den unterschiedlichen primären und sekundären Aminen auf an sich herkömmliche Weise gaschromatographisch ermittelt werden.

Die tert. Amine werden mit den Abfangreagenzien vor ihrer Oxidation zu den Aminoxiden in der Regel während einer Zeitdauer von 0,25 bis 3 h, vorzugsweise 0,5 bis 2 h und besonders bevorzugt von 1 bis 1,5 h bei Temperaturen von 0 bis 150°C, vorzugsweise bei 20 bis 80°C und besonders bevorzugt bei Raumtemperatur inkubiert. Die Inkubation kann bei Atmosphärendruck oder unter erhöhtem Druck, insbesondere dem Eigendruck des Reaktionssystems, durchgeführt werden, bevorzugt wird bei Atmosphärendruck inkubiert. Mit besonders reaktiven Abfangreagenzien, wie Ketenen oder Säurechloriden, und bei Anwendung erhöhter Temperaturen ist es möglich, die für eine möglichst vollständige Derivatisierung der unerwünschten Amine erforderliche Inkubationszeit weiter zu verkürzen, es kann jedoch aus wirtschaftlichen Gründen zweckmäßiger sein, bei niedrigeren Temperaturen und längeren Inkubationszeiten zu inkubieren.

Nach der Inkubation mit dem jeweils angewandten Abfangreagenz zur Derivatisierung der primären und sekundären Amine kann der Reaktionsmischung ohne weitere Aufarbeitung das Oxidationsmittel zum Zwecke der Umwandlung der tert. Amine in tert. Aminoxide zugefügt werden. Für die Oxidation der tert. Amine in die entsprechende Aminoxide können die für diesen Zweck an sich üblichen Oxidationsmittel, in der Regel wird eine wäßrige Wasserstoffperoxidlösung verwendet, und Oxidationsverfahren benutzt werden, beispielsweise die der DE-A 36 18 352 und der DE-A 16 94 048. Auch die Aufarbeitung des Reaktionsproduktes kann nach den an sich üblichen Methoden, beispielsweise den der vorstehend genannten Schriften, erfolgen.

Das erfindungsgemäße Verfahren kann grundsätzlich bei der Herstellung aller tert. Aminoxide aus tert. Aminen angewandt werden. Insbesondere ist das erfindungsgemäße Verfahren zur Herstellung von tert. Aminoxiden aus tert. aliphatischen Aminen der Formel XVIII 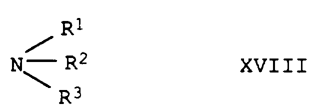 geeignet, in denen die Reste R¹, R und R³ gleich oder verschieden sein können und für C₁- bis C₂₀-Alkyl-, vorzugsweise C₁- bis C₁₀-Alkyl- und besonders bevorzugt für C₁- bis C₆-Alkylgruppen, für C₃- bis C₈-Cycloalkyl-, vorzugsweise für C₅-C₆-Cycloalkylgruppen, für C₇- bis C₁₂-Aralkylgruppen, vorzugsweise die Benzylgruppe sowie für gegebenenfalls substituierte Phenyl- oder Naphthylgruppen stehen können und in denen die Reste R¹ und R alternativ gemeinsam zu einem 3- bis 10-gliedrigen, cycloaliphatischen Ring verbunden sein können, der als Heteroatom zusätzlich ein Sauerstoffatom enthalten kann. Besonders bevorzugt werden solche cycloaliphatischen, tert. Amine verwendet, in denen R¹ und R gemeinsam mit dem Stickstoffatom einen 5- bis 7-gliedrigen, cycloaliphatischen Ring bilden und R³ eine C₁- bis C₁₀-Alkyl-, vorzugsweise eine C₁- bis C₄-Alkylgruppe ist. Als Beispiele für derartige besonders bevorzugte Ausgangsverbindungen seien N-Methylpyrrolidin, N-Ethylpyrrolidin, N-n-Propylpyrrolidin, N-n-Butylpyrrolidin, N-Methylpiperidin, N-Ethylpiperidin, N-n-Propylpiperidin, N-n-Butylpiperidin, N-Methylmorpholin, N-Ethylmorpholin, N-n-Propylmorpholin, N-n-Butylmorpholin sowie N-Methylazacycloheptan genannt. Weiterhin können als tert. Amine N,N'-C₁- bis C₄-Dialkylpiperazine sowie Chinuclidin vorteilhaft als Ausgangsmaterialien lien zur Herstellung von tert. Aminoxiden nach dem erfindungsgemäßen Verfahren verwendet werden. Die genannten tertiären Amine sind im Handel erhältlich oder können aus den entsprechenden Alkoholen durch deren reduktive Aminierung mit Ammoniak z.B. nach den Verfahren gemäß DE-A 19 53 263 und US-A 5 002 922 hergestellt werden.

Mit dem erfindungsgemäßen Verfahren können mit minimalem Aufwand Lösungen tertiärer Aminoxide erhalten werden, deren Nitrosamingehalt, bestimmt durch Gaschromatographie-Massenspektrometrie-Kupplung oder nach dem Verfahren von Krull et al., Anal. Chem. 51, 1706 (1979) unter 50 ppb liegt.

### Beispiele

### Vergleichsbeispiel

Herstellung von N-Methylmorpholin-N-oxid (NMO) aus N-Methylmorpholin (NMM) mit einem Gehalt von 0,3 Gew.-% primären und sekundären Aminen.

In einer 500 ml Rührapparatur wurden unter einer Stickstoffatmosphäre 101 g NMM (1,0 mol) vorgelegt und unter Rühren während eines Zeitraums von 2 h 102,4 g Wasserstoffperoxid in Form einer 30 gew.-%igen, wäßrigen Lösung (0,9 mol) bei 70°C zudosiert. Nach beendeter Wasserstoffperoxid-Zugabe wurde die Reaktionsmischung noch 7 h bei dieser Temperatur gerührt. Anschließend wurden bei einer Temperatur von 60°C und einem Druck von 100 mbar ca. 20 ml überschüssiges wäßriges NMM abdestilliert. Als Destillationssumpf blieb eine ca. 57 gew.-%ige, wäßrige NMO-Lösung zurück.

Analysendaten der erhaltenen NMO-Lösung:
Restgehalt an NMM: < 0,1 Gew.-%
Nitrosamingehalt: 3100 ppb

Der Gehalt der NMO-Lösung an NMM und NMO wurde durch Säure-Base-Titration mit potentiometrischer Detektion bestimmt, ihr Nitrosamin-Gehalt wurde gaschromatographisch/massenspektrometrisch, nach der vorherigen Abtrennung des NMM's und NMO's mittels eines sauren Ionenaustauschers, gemessen. Diese Analysenmethoden wurden bei allen NMO-Proben angewandt.

### Beispiel 1 (erfindungsgemäß)

Eine ca. 57 gew.-%ige, wäßrige NMO-Lösung wurde aus NMM, dessen Gehalt an primären und sekundären Aminen destillativ auf 0,02 Gew.-% abgesenkt worden war, nach dem Verfahren des Vergleichsbeispiels hergestellt.

Analysendaten:
Restgehalt an NMM: < 0,1 Gew.-%
Nitrosamingehalt: < 50 ppb

### Beispiel 2

Wie im Vergleichsbeispiel wurden 101 g NMM, das einen Gehalt von primären und sekundären Aminen von 0,3 Gew.-% hatte, bei Raumtemperatur vorgelegt und unter Rühren mit 0,65 g (4,5 mmol) Acetanhydrid versetzt. Es wurde 1 h inkubiert und anschließend, wie im Vergleichsbeispiel beschrieben, zu NMO oxidiert und aufgearbeitet.

Die so erhaltene 57 gew.%ige NMO-Lösung hatte die folgenden Analysendaten:
Restgehalt an NMM: < 0,1 Gew.-%
Nitrosamingehalt: < 20 ppb

In weiteren Versuchen gemäß diesem Verfahren wurden anstelle des Acetanhydrids jeweils 4,5 mmol eines der Abfangreagenzien p-Toluolsulfonsäurechlorid, Acetylchlorid, gasförmiges Keten, Methansulfonsäurechlorid und Dimethoxyphosphorylchlorid der Formel XIX 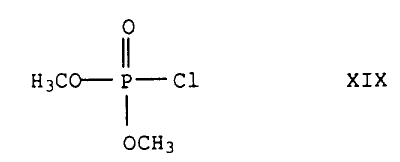 eingesetzt. Bei Rest-NMM-Gehalten von jeweils < 0,1 Gew.-% lag der Nitrosamingehalt bei allen derart hergestellten NMO-Lösungen unter 20 ppb.

## Patentansprüche

1. Verfahren zur Herstellung tertiärer Aminoxide mit einem Nitrosamingehalt unter 50 ppb durch die Umsetzung eines tertiären Amins mit einer wäßrigen Wasserstoffperoxidlösung, dadurch gekennzeichnet, daß man als Ausgangsmaterial tertiäre Amine verwendet, deren Gehalt an primären und sekundären Aminen insgesamt nicht mehr als 0,05 Gew.-% beträgt.

2. Verfahren nach Anspruch 1, dadurch gekennzeichnet, daß man dem tertiären Amin vor der Umsetzung ein Abfangreagenz für primäre und sekundäre Amine zusetzt.

3. Verfahren nach Anspruch 1, dadurch gekennzeichnet, daß man dem tertiären Amin vor seiner Umsetzung als Abfangreagenz ein Abfangreagenz aus der Klasse der Acylhalogenide, Carbonsäureanhydride, Ketene, Sulfonylhalogenide oder Phosphorylhalogenide zusetzt.

## Claims

1. A process for preparing tertiary amine oxides with a nitrosamine content of below 50 ppb by reacting a tertiary amine with an aqueous solution of hydrogen peroxide, which comprises using as starting material tertiary amines whose total content of primary and secondary amines is not more than 0.05% by weight.

2. A process as claimed in claim 1, wherein a scavenger for primary and secondary amines is added to the tertiary amine before the reaction.

3. A process as claimed in claim 1, wherein the scavenger added to the tertiary amine before its reaction is from the class of acyl halides, carboxylic anhydrides, ketenes, sulfonyl halides or phosphoryl halides.

## Revendications

1. Procédé de préparation d'oxydes d'amines tertiaires possédant une teneur en nitrosoamine inférieure à 50 ppb par la réaction d'une amine tertiaire avec une solution aqueuse de peroxyde d'hydrogène, caractérisé en ce que l'on utilise, à titre de matière de départ, des amines tertiaires dont la teneur en amines primaires et secondaires n'est, au total, pas supérieure à 0,05% en poids.

2. Procédé suivant la revendication 1, caractérisé en ce que, avant la réaction, on ajoute un réactif de prise pour les amines primaires et secondaires à l'amine tertiaire.

3. Procédé suivant la revendication 1, caractérisé en ce que, avant sa réaction, on ajoute à l'amine tertiaire, à titre de réactif de prise, un réactif de prise appartenant à la classe des halogénures d'acyle, des anhydrides d'acides carboxyliques, des cétènes, des halogénures de sulfonyle, ou des halogénures de phosphoryle.
